# EUROPÄISCHE PATENTANMELDUNG

(11) **EP 1 579 827 A2**
(43) Veröffentlichungstag der Anmeldung: **28.09.2005**
(21) Anmeldenummer: 04024807.2
(22) Anmeldetag: 19.10.2004
(51) Int. Cl.: A61F 2/24

(54) **Kardiovaskuläres Implantat, Verfahren und Vorrichtung zur Herstellung und Bereitstellung für die Chirurgie**

(30) Priorität: 24.10.2003 DE 10350287
(71) Anmelder: DEUTSCHE INSTITUTE FÜR TEXTIL- UND FASERFORSCHUNG STUTTGART Stiftung des öffentlichen Rechts, 73770 Denkendorf (DE); Meiser, Bruno M., D-80803 München (DE); Gulbins, Helmut, Dr., D-89134 Blaustein-Herrlingen (DE); Reichart, Bruno, Prof. Dr., D-82319 Leutstetten (DE)
(72) Erfinder: Dauner, Martin, 73728 Esslingen (DE); Doser, Michael, 70794 Filderstadt (DE); Kottler, Reinhard, 72660 Beuren (DE); Planck, Heinrich, 72622 Nürtingen-Zizishausen (DE); Gulbins, Helmut, 89134 Blaustein-Herrlingen (DE); Meiser, Bruno, 80803 München (DE); Reichart, Bruno, 82319 Leutstetten (DE)
(74) Vertreter: Patentanwälte Ruff, Wilhelm, Beier, Dauster & Partner

(57) **Zusammenfassung**

Es wird ein kardiovaskuläres Implantat zur Verwendung in der Chirurgie bereitgestellt, insbesondere eine Herzklappe mit Segeln, gebildet im Wesentlichen aus bioverträglichem synthetischem Polymermaterial in Form einer dreidimensionalen Struktur, bei dem das Polymermaterial unter physiologischen Bedingungen im Wesentlichen nicht resorbierbar ist, das Implantat mindestens an einem Teil seiner Oberfläche als mikroporöses Vlies aus Mikrofasern des Polymermaterials gebildet ist, und die eine Besiedelung mit Zellen ermöglichen.

## Beschreibung

Die vorliegende Erfindung betrifft ein kardiovaskuläres Implantat, ein Verfahren zu seiner Herstellung, eine Vorrichtung zu seiner Herstellung und seine Bereitstellung für die Chirurgie.

In der Chirurgie werden Implantate zur Unterstützung, zum völligen oder teilweisen Ersatz erkrankter oder traumatisierter Körperorgane eingesetzt. Solche Implantate oder Prothesen können unter Verwendung von Metallen, synthetischen Kunststoffen, Biopolymeren oder biologischem Material von Tier oder Mensch ausgebildet sein.

Probleme ergeben sich bei Implantaten dadurch, dass der Körper des Empfängerpatienten das Implantat als Fremdkörper erkennt und Abwehrmechanismen aktiviert, die bis zur Funktionsunfähigkeit und Abstoßung des Implantats führen können.

Implantate aus Metall, Kunststoff oder Verbundmaterial sind mechanisch beständig, jedoch fördern ihre Oberflächen die Bildung von Blutgerinnseln, so dass die Patienten auf Dauer blutverdünnende Medikamente einnehmen müssen, die wiederum zu Blutungskomplikationen führen können.

Aus biologischem Material, beispielsweise von Schweinen, Rindern oder von menschlichen Spendern gewonnene Implantate zeigen keine thromboembolischen Komplikationen. Jedoch können bei Implantation von nicht vom Patienten selbst stammendem biologischem Gewebe immunologische Reaktionen auftreten, die dazu führen, dass das Implantat innerhalb von 10 bis 20 Jahren nach dem Einsetzen degeneriert und eine erneute Operation des nun ebenfalls betagteren Patienten erforderlich ist.

Ansätze durch Zellbesiedlung von resorbierbaren Stützstrukturen einen Ersatz für Blutgefäße oder Herzklappen zu erzeugen, scheitern aufgrund der unzureichenden Festigkeit des neugebildeten Gewebes.

Von besonderer Bedeutung ist eine zuverlässige, langanhaltende Funktionsfähigkeit ohne Risiko weiterer gesundheitlicher Beeinträchtigungen bei Implantaten im Bereich des Herz-Kreislaufsystems, wie beispielsweise Gefäßprothesen oder Herzklappen.

Es stellt sich daher die Aufgabe, ein Implantat zur Verfügung zu stellen, das die Probleme und Nachteile von Implantaten aus dem Stand der Technik überwindet, im Patienten implantiert dauerhaft und zuverlässig funktionsfähig ist sowie einfach und kostengünstig nach medizintechnischen Verfahrensweisen herzustellen ist.

Diese Aufgabe wird gelöst durch ein kardiovaskuläres Implantat zur Verwendung in der Chirurgie, insbesondere Herzklappe mit Segeln, gebildet im Wesentlichen aus bioverträglichem synthetischem Polymermaterial in Form einer dreidimensionalen Struktur, dadurch gekennzeichnet, dass das Polymermaterial unter physiologischen Bedingungen im Wesentlichen nicht resorbierbar ist, das Implantat mindestens an einem Teil seiner Oberfläche als mikroporöses Vlies aus Mikrofasern des Polymermaterials ausgebildet ist, die eine Besiedelung mit Zellen ermöglichen. Hierdurch wird eine dauerhafte Unterstützung des Gewebes erreicht, die u. a. im Hochdruckbereich (arteriellen Bereich) erforderlich ist.

Das erfindungsgemäße Polymermaterial zeichnet sich mit Vorteil dadurch aus, dass die poröse Struktur das Implantats einen Stoffwechselaustausch und insbesondere die Haftung von Zellen begünstigen kann. Die Porengröße der Mikroporen kann bevorzugt im Bereich von 0,1 bis 20 µm, insbesondere 1 bis 8 µm liegen.

Mit Vorteil kann das Polymermaterial mindestens teilweise ein solches auf Basis von Polyurethan (PUR) sein. Insbesondere kann das Polyurethan ein lineares Polyurethan sein. Bevorzugt kann das Polyurethan als Kettenverlängerer Siliconpolyurethane enthalten. Als Beispiele für erfindungsgemäß zu verwendende Polyurethane sind segmentierte aromatische und aliphatische Polyurethane zu nennen, die Hartsegmente ausgewählt aus Methylen-bis-4-cyclohexylisocyanat und 4,4'-Methylenbisphenyldiisocyanat, Weichsegmente ausgewählt aus Polytetramethylenetherglycol, Polycarbonat, Polytetramethylenoxid/Polydimethylsiloxan und Polycarbonat/Polydimethylsiloxan sowie Kettenverlängerer ausgewählt aus Ethylendiol und 1,4-Butandiol umfassen. Die Molekulargewichte können 50 kD bis 300 kD liegen. Scherviskositäten für beispielhafte Polymere gemäß der Erfindung können im Bereich von 66 mPas bei einer Lösungskonzentration von 5 % bis zu 1294 mPas bei einer Lösungskonzentration von 10 % betragen.

In einer anderen Ausführungsform der Erfindung kann das Polymermaterial mindestens teilweise auf Basis von Polyethylenterephthalat (PET) sein. Als weitere erfindungsgemäß einsetzbare Polymere sind Polyurethan (PUR), thermoplastisches Polyurethan (TPU), thermoplastisches Elastomer (TPE) und Polyetherblockamid (PEBA) zu nennen. Mit besonderem Vorteil kann sich das Polymermaterial der erfindungsgemäßen Herzklappe durch seine hohe Elastizität auszeichnen. Ebenso kann sich das Polymermaterial der erfindungsgemäßen Herzklappe durch seine hohe Flexiblität auszeichnen.

In einer besonderen Ausführungsform der Erfindung kann das Polymermaterial teilweise unter physiologischen Bedingungen resorbierbar sein.

Mit Vorteil kann die Struktur, insbesondere im Bereich der Herzklappensegel, aus Polymerfasermaterial als textiles Nonwoven, insbesondere als Sprühvlies ausgebildet sein. In einer anderen Ausführungsform kann das Polymerfasermaterial als Nonwoven nach einem elektrostatischen Vliesherstellungsverfahren gebildet sein. In noch einer anderen Ausführungsform kann das Polymerfasermaterial als Nonwoven nach einer Melt-Blow-Technik hergestellt sein. Als alternative Ausführungsform kann das Polymerfasermaterial unter Anwendung einer Kombination der zuvor genannten Vliesherstellungstechniken zu einem Nonwoven ausgebildet sein.

Bevorzugt kann die Vliesstruktur Oberflächenrauhigkeit aufweisen.

Im erfindungsgemäßen Implantat kann das textile Material eine Dicke von 10 bis 500 µm, insbesondere 30 bis 70 µm aufweisen. Bevorzugt können die Mikrofasern eine Dicke von 0,1 bis 10 µm aufweisen. Außerdem können die Mikrofasern eine Länge von 5 bis 25 mm aufweisen. Erfindungsgemäß bevorzugt kann das Vlies eine Porengröße im Bereich der Faserdicke aufweisen. Mit Vorteil können die Mikrofasern zwischen Verbindungsstellen eine Länge von 0,1 bis 10 µm aufweisen. Bevorzugt kann eine gegenseitige Verbindung der Mikrofasern eine Verklebung, insbesondere eine Verschweißung sein.

In Weiterbildung der Erfindung kann ein Implantat zur Verfügung gestellt sein in Form einer Herzklappenprothese umfassend einen rohrförmigen Abschnitt als Stumpf eines Herzblutgefäßes, insbesondere Aortenstumpf, der zum Annähen an ein Endstück eines natürlichen Blutgefäßes ausgebildet ist, wobei an den Stumpf drei, eine Herzklappe bildende Segel angeordnet sind, die beweglich sind und miteinander ventilartig zusammenwirken, um bei Blutdurchfluss zu öffnen und zu schließen. Auf diese Weise kann eine vollständige Herzklappenprothese mit Klappensegeln, Bulbi und Sinus zur Verfügung gestellt werden.

Mit Vorteil sind die Herzklappensegel einer natürlichen Herzklappe nachgebildet. Bevorzugt können die Herzklappensegel eine zur Mitte der Herzklappe hin abnehmende Dicke aufweisen. Die Konstruktion der Segel und des das Blutgefäß bildenden Abschnitts sind bevorzugt so ausgewählt, dass eine Vaskularisierung begünstigt wird. Mindestens die Segel bestehen vorzugsweise vollständig aus Vliesmaterial.

Bei einer solchen Herzklappenprothese können die Herzklappensegel aus flächigem Vlies gebildet sein. Insbesondere können die Herzklappensegel gewölbt ausgebildet sein. Mit Vorteil können die Herzklappensegel thermisch und/oder bei der Herstellung geformt sein. Die Herzklappensegel im erfindungsgemäßen Implantat können eine Dicke von 10 bis 500 µm aufweisen. Insbesondere kann das Implantat im Bereich des Gefäßabschnitts eine Wanddicke von 1 bis 3 mm aufweisen.

In einer Ausführungsform der Erfindung können die Herzklappensegel separat hergestellt und am rohrförmigen Abschnitt der Prothese befestigt, insbesondere eingeklebt sein. Die Segel können mit Flüssigkleber, insbesondere Polyurethankleber eingeklebt sein. Bevorzugt können die Segel mit Polymer aus der Vliesherstellung eingeklebt sein. In einer anderen Ausführungsform der Erfindung können die Herzklappensegel separat hergestellt und am rohrförmigen Abschnitt der Prothese befestigt, insbesondere angeschmolzen sein. Eine solche durch Schmelzen des Polymermaterials ausgebildete Befestigung kann durch Ultraschallschweißen erreicht werden.

In einer anderen Ausführungsform der Erfindung kann das Implantat, insbesondere die Herzklappenprothese, speziell der rohrförmige Abschnitt mit den Segeln einstückig ausgebildet sein.

Gemäß der Erfindung kann das Implantat, insbesondere die Herzklappenprothese direkt in dreidimensionaler Form hergestellt werden. Hierzu sind beispielsweise elektrostatische Verfahrensweisen, Umformen von Vorformen und Tempern zu nennen. In einer Ausführungsform kann das Implantat mindestens teilweise unter Verwendung einer Sprühtechnik hergestellt sein. In einer anderen Ausführungsform kann das Implantat mindestens teilweise nach einer bekannten elektrostatischen Spinntechnik hergestellt sein. Das Implantat kann unter Verwendung einer Kombination von Verfahrenstechniken zum Sprühen und Spinnen von Mikrofasern hergestellt sein. In Weiterbildung kann die erfindungsgemäße Herzklappenprothese mit einem Nahtring versehen sein.

In einer vorteilhaften Ausführungsform der Erfindung können verschiedene Teile des Implantats, insbesondere die Herzklappensegel und der rohrförmige Abschnitt, in unterschiedlichen textilen Konstruktionen und/oder aus unterschiedlichen Materialien ausgebildet sein. Bevorzugt können die Herzklappensegel als textile vliesartige Membran ausgebildet sein. In einer Ausführungsform kann der rohrförmige Blutgefäßabschnitt als textiles Gewirk ausgebildet sein. In einer anderen Ausführungsform kann der rohrförmige Blutgefäßabschnitt als textiles Gewebe ausgebildet sein. Bevorzugt ist, die gesamte Oberfläche des Implantats als Vlies auszubilden, insbesondere das gesamte Implantat selbst.

In einer vorteilhaften Ausführungsform können die Herzklappensegel und. der rohrförmige Abschnitt in unterschiedlichen Porengrößen ausgebildet sein. Die unterschiedlichen Porengrößen können bei der Zellbesiedelung von Vorteil sein. Im Falle der Herzklappensegel ist die Oberflächenbesiedelung zu begünstigen. Im Falle des Blutgefäßabschnitts ist das Eindringen von Zellen zu begünstigen.

In einer alternativen Ausführungsform können die Herzklappensegel und der rohrförmige Abschnitt aus unterschiedlichen Materialien hergestellt sein. Die Materialien können sich vorteilhaft in ihren physikalischen und/oder mechanischen Eigenschaften unterscheiden, wie beispielsweise in der Flexibilität und Elastizität. Auf diese Weise können die Forderungen an eine optimale Funktion der Herzklappenprothese unter physiologischen Bedingungen im Patientenkörper erfüllt werden.

In einer alternativen Ausführungsform können die Herzklappensegel an eine gewebte oder gewirkte oder durch Melt-Blow-Technik hergestellte Gefäßprothese, z. B. aus PET oder PVDF (Polyvinylidenfluorid), angearbeitet sein. Insbesondere kann eine Gefäßprothese nach dem Stand der Technik als rohrförmiger Abschnitt verwendet werden.

Mit Vorteil kann das Implantat, insbesondere, der rohrförmige Abschnitt der Herzklappenprothese mit einer Verstärkung, insbesondere in Form einer textilen Konstruktion versehen sein. Bevorzugt kann die Verstärkung in Form eines Geflechts ausgebildet sein. In einer anderen Ausführungsform kann die Verstärkung als Fasergelege radial und um den Umfang der Segel ausgebildet sein. Mit Vorteil kann die Verstärkung aus monofilen, in der Hauptverformungsrichtung angeordneten Fäden ausgebildet sein. Ferner kann die Verstärkung auf Basis von Polyethylenterephthalat (PET) ausgebildet sein.

Mit besonderem Vorteil ist das erfindungsgemäße Implantat zur Besiedelung mit lebenden Zellen, insbesondere humanen Zellen geeignet. Eine solche Zellbesiedlung kann einschichtig oder mehrschichtig ausgebildet sein. Auf diese Weise kann es als flexibles Gerüst zur Herstellung eines mit Zellen besiedelten, biohybriden Implantats dienen. Die vliesartige Oberflächengestaltung und die Materialwahl zur Ausbildung eines kardiovaskulären Implantats ermöglichen ein leichtes Anhaften von Zellen auf den Oberflächen des Implantats. Es kann sich eine gut anhaftende Zellschicht ausbilden. Gegebenenfalls und bevorzugt können mehrere Zellschichten übereinandergelagert sein, insbesondere können sich die Zellschichten durch unterschiedliche Zellarten voneinander unterscheiden. Ein Einwachsen von Zellen in das Implantat kann durch entsprechende Wahl der mikroporösen Struktur begünstigt oder beeinträchtigt sein. Durch Bereitstellen eines Gerüstes bzw. Rohlings für eine Herzklappenprothese ist es möglich, auf einfache Weise vor Ort ein biohybrides Implantat besiedelt mit Zellen des betroffenen Patienten selbst herzustellen.

In einer besonderen Ausführungsform der Erfindung kann das Implantat als biohybrides kardiovaskuläres Implantat ausgebildet sein. Das erfindungsgemäße Implantat kann sich dadurch auszeichnen, dass es mindestens teilweise, insbesondere mehrschichtig, mit humanen Zellen besiedelt ist. Angesiedelte Zellen können auf dem Vlies verankert sein. Insbesondere können die angesiedelten Zellen als flächiger Zellrasen ausgebildet sein. Bevorzugt können die Zellen geschichtet sein. In einer bevorzugten Ausführungsform des erfindungsgemäßen Implantats können angesiedelte Zellen die dem Blut zugewandte Oberfläche als flächiger Zellrasen überziehen.

Vorteilhaft können die angesiedelten Zellen aus der Gruppe der Fibroblasten, glatten Muskelzellen und Endothelzellen ausgewählt sein. In Weiterbildung können angesiedelte Zellen auch aus der Gruppe der differenzierten Stammzellen und klonierten Zellen ausgewählt sein. Erfindungsgemäß bevorzugt können Zellen des Implantats, die mit dem Blutstrom des Patienten in Kontakt kommen, Endothelzellen sein. Insbesondere können die Endothelzellen auf einer Fibroblasten und/oder glatte Muskelzellen enthaltenden Zellschicht angesiedelt sein. Ein intaktes Endothel beeinflusst das Blutgerinnungsverhalten, so dass sogar bereits aktivierte Gerinnungsparameter wieder deaktiviert werden. Die im erfindungsgemäßen Implantat unter den Endothelzellen liegende Zellschicht kann als Haftschicht wirken. Die Haftung des Endothels auf dem Untergrund ist von entscheidender Bedeutung für seine Funktionsfähigkeit. Bei unzureichender Haftung besteht die Gefahr, dass die Zellen mit dem Blutstrom abgeschwemmt werden und so eine körperfremde Implantatoberfläche freigelegt wird. Mit Vorteil kann der angesiedelte Zellrasen eine hohe Scherfestigkeit aufweisen, das heißt, der angesiedelte Zellrasen kann unter den physiologisch auftretenden Scherkräften stabil bleiben.

Das Implantat gemäß der Erfindung kann sich dadurch auszeichnen, dass der angesiedelte Zellrasen im Blut befindliche Gerinnungsparameter deaktiviert. Möglicherweise als Reaktion auf die Implantierung gebildete Gerinnungsstoffe können so in ihrer Wirkung gehemmt werden. Für den Patienten sind damit die mit einer Blutgerinnung, Blutverdickung und Thrombusbildung einhergehenden Risiken nach einem kardiovaskulären Eingriff wesentlich verringert. Die Einnahme von gerinnungshemmenden und blutverdünnenden Medikamenten kann auf ein Mindestmaß beschränkt bleiben oder es kann ganz darauf verzichtet werden.

Bevorzugt können die angesiedelten Zellen aus vom Implantatempfänger stammenden Zellen kultiviert sein. Auf diese Weise können Fremdkörperreaktionen beim behandelten Patienten minimiert werden. Das aus einer Vorform aus synthetischem Polymermaterial gebildete Implantat ist unter einer Zellbesiedelung mit körpereigenen Zellen des Patienten verborgen, so dass die Immunabwehr des Körpers am Implantat "eigenes" und kein "fremdes" Material erkennt und Abwehrreaktionen unterlässt. Für den Patienten ist auf diese Weise der Vorteil einer langanhaltenden Funktionsfähigkeit des Implantats gegeben. Gleichzeitig kann die Verabreichung von Medikamenten zur Unterdrückung von Fremdkörper- und Abwehrreaktionen auf ein Mindestmaß beschränkt oder ganz darauf verzichtet werden.

Die vorliegende Erfindung betrifft ferner ein Verfahren zur Herstellung eines biohybriden Implantats, gebildet im Wesentlichen aus bioverträglichem synthetischem Polymermaterial in Form einer dreidimensionalen Struktur, dadurch gekennzeichnet, dass eine Vorform des Implantats aus synthetischem Polymermaterial mit humanen Zellen besiedelt wird, wobei zunächst Fibroblasten und/oder glatte Muskelzellen, angesiedelt werden, dann vorzugsweise nach einer Ruhephase zur Verankerung der ersten Zellschicht, in einer Oberflächen-Zellschicht Endothelzellen angesiedelt werden.

Bevorzugt können Endothelzellen, Fibroblasten und glatte Muskelzellen aus explantierten Stücken der Vena saphena magna des zu behandelnden Patienten gewonnen und in spezifischen Zellmedien unter Zusatz von humanem Serum des Patienten kultiviert werden. Die Verfahrensweise der Zellgewinnung und Zellkultivierung ist den Fachleuten bekannt, so dass hier auf eine Beschreibung verzichtet wird.

Gemäß der Erfindung kann ein kardiovaskuläres Implantat sowohl auf der Innenseite wie auf der Außenseite stabil mit humanen Zellen besiedelt werden. Insbesondere kann die Innenseite vollständig mit Endothelzellen ausgekleidet werden. Ferner kann die Außenseite des Implantats, insbesondere der Herzklappenprothesen, mit glatten Muskelzellen und Fibroblasten besiedelt werden. Durch die Zellschicht wird die Wand des Implantats im mechanisch besonders belasteten Bereich zusätzlich gefestigt und vor Entzündungsreaktionen des Patientenkörpers geschützt. Bevorzugt tragen die Segel nur eine dünne Fibroblastenschicht mit einer konfluenten Endothelzellenschicht darauf.

In Versuchen mit dem erfindungsgemäßen Implantat wurde gefunden, dass eine Vorbesiedelung mit Fibroblasten oder einer Mischkultur aus Fibroblasten und glatten Muskelzellen selbst bei einem kleinen Endothelzellverlust die Blutgerinnung weit weniger aktiviert, als dies bei freiliegender körperfremder Oberfläche der Fall ist. Dies ist darauf zurückzuführen, dass kleinere Defekte in der Endothelzellschicht auf einem derartig physiologischen Untergrund ausgeglichen, sozusagen repariert werden.
Die Zellen können bei Bedarf aus Vorläufer- oder Stammzellen gewonnen werden.

Mit Vorteil kann die Herzklappe während und/oder nach ihrer Herstellung mit rohr- bzw. schlauchartigen Verbindungsstücken verbunden werden, die zum Verbinden mit dem Herzen und dem Aortenbogen dienen.

Die Erfindung stellt auch eine Vorrichtung zur Herstellung eines biohybriden Implantats, insbesondere zur Durchführung des erfindungsgemäßen Verfahrens zur Verfügung, die gekennzeichnet ist durch einen zur Zellbesiedelung einer Vorform eines chirurgischen Implantats aus synthetischem Polymermaterial eingerichteten Inkubator zur Aufnahme des Zellmediums, insbesondere einer Zellsuspension, der um mindestens eine Achse, vorzugsweise um mehrere Achsen drehbar ist, und ein darin aufnehmbares Gestell zur Aufnahme und Fixierung des mit Zellen zu beschichtenden Implantats aufweist.

Um die Zellen homogen auf dem entsprechenden Implantat ansiedeln zu können, handelt es sich bei der Vorrichtung um ein rundes Gefäß, das um seine eigene Achse rotiert werden kann. Das zu beschichtende Implantat wird in einem als Haltegestell ausgebildeten Inlay fixiert. In einer Ausführungsform kann der Inkubator beispielsweise 50 bis 200 ml Zellmedium aufnehmen und 5 bis 20 ml Zellsuspension mit 10⁶ bis 10⁸ Zellen. Mit Hilfe einer Rotationsmaschine kann der Inkubator während mehrerer Stunden gedreht werden. Die Rotationsgeschwindigkeiten können zwischen weniger als einer und 30 Umdrehungen pro Minute liegen, bevorzugt sind 1 bis 10 Umdrehungen pro Minute. Mit Vorteil können sich Rotationsphasen mit Standphasen von etwa 10 bis 60 Minuten, insbesondere 15 bis 45 Minuten Dauer abwechseln. Die Standphasen ermöglichen ein Absetzen der Zellen auf dem Untergrund, der Implantatoberfläche, gemäß der Schwerkraft. Während der Rotationsphasen werden Zellen, die noch nicht adhärent sind, wieder in Suspension gebracht und im Inkubator verteilt. Die Rotationsgeschwindigkeit wird so gewählt, dass nach jeder Rotation ein anderer Teil der Implantatoberfläche nach oben ausgerichtet und somit für die Zellbesiedelung exponiert wird. Dies wird dadurch erreicht, dass die jeweils letzte Umdrehung eines Rotationszyklus nicht vollständig durchgeführt wird, sondern nur 351 bis 359° beträgt.

In einer anderen Ausführungsform der erfindungsgemäßen Vorrichtung kann sich der Inkubator zusätzlich im Uhrzeigersinn drehen. Hierdurch bewegt sich das Inlay bei jeder 180°-Drehung, so dass es bei jeder 360°-Rotation zu einem kleinen Fluss des Mediums mit Zellsuspension durch das zu besiedelnde dreidimensionale Implantat kommt.

Umdrehungen und Standzeiten können je nach Zellart, Zelldichte und gewünschter Zellschichtdicke gewählt werden, um auf diese Weise eine gewünschte Verteilung der Zellen auf dem Substrat zu erreichen.

Zur Überprüfung der Beständigkeit der Zellschichten gegen Scherkräfte wird ein In-Vitro-Test in einer Perfusionskammer durchgeführt, wobei verschiedene Zustände, wie sie im Körper eines Patienten vorkommen, standardisiert getestet werden können. Die zu prüfende Herzklappenprothese wird mit zwei Gefäßprothesen verlängert in der Perfusionskammer fixiert, so dass sie sich frei im Prüfmedium, Zellmedium oder blutgruppenkompatiblem Blut, befindet. Durch eine pulsatile Pumpe wird das Perfusat in physiologischer Richtung durch die Prothese befördert, wobei eine Frequenz zwischen 60 und 120 Pulsschlägen pro Minute eingestellt werden kann. Durch eine zweite Pumpe wird proximal von der Klappe kontinuierlich mit gerigner Fördermenge von etwa 100 ml/min Volumen abgesaugt, wodurch Klappenschluss verursacht wird. Die Bewegungen der Klappensegel bedeuten eine besondere Belastung für die Zellbeschichtung. Die Klappen werden auf Druckwerte zwischen 100 und 200 systolisch sowie 30 bis 80 diastolisch getestet. Über eine vor die Klappe eingeführte Kamera kann während des Versuchs das Spiel der Klappensegel dokumentiert werden.

Die vorliegende Erfindung umfasst auch die Bereitstellung eines chirurgischen Implantats zur Verwendung als Gefäßprothese in der kardiovaskulären Chirurgie. Mit besonderem Vorteil können schmallumige Bypass-Gefäße mit Durchmessern im Bereich von bis zu 5 Millimetern aus dem erfindungsgemäßen Implantat eingesetzt werden. Bei bekannten Prothesen für den aorto-koronaren Bypass mit kleinem Gefäßdurchmesser wirkt die fremde Oberfläche thrombogen, was zu frühzeitiger Aktivierung der Gerinnungskaskade und zu thrombotischem Verschluss des Gefäßes führt. Die erfindungsgemäß mittels Patientenzellen ausgebildete innere Endotheloberfläche kann eine Thrombenbildung und somit Verschluss der implantierten Gefäße verhindern.

Für eine weitere Verwendung kann das Implantat gemäß der Erfindung als Herzklappenprothese in der Herzchirurgie bereitgestellt werden. Mit besonderem Vorteil kann die erfindungsgemäße Herzklappenprothese als Implantat im Hochdruckkreislauf der linken Herzhälfte verwendet werden. Die Implantation von außen und innen mit autologen Zellen besiedelten Herzklappenprothesen ist für den Patienten mehrfach von Vorteil. Da die innere Oberfläche, die mit dem Blutstrom in Kontakt tritt, aus patienteneigenen Endothelzellen besteht und somit die Bildung von Blutgerinnseln verhindert, ist eine gerinnungshemmende Medikation überflüssig. Zusätzlich sind keine immunologischen Reaktionen, wie Abstoßung zu erwarten, da nur patienteneigene Zellen mit dem Immunsystem des Patienten in Kontakt kommen.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

### Beispiele

### Beispiel 1

### Verwendung der Besiedelungsvorrichtung zur Zellbesiedelung eines Implantats

Der Inkubator mit dem darin fixierten zu beschichtenden Implantat wird mit 100 ml Zellmedium und 10 ml Zellsuspension, sie enthält 10⁶ bis 10⁸ Zellen, beschickt und für 12 bis 24 Stunden in eine Rotationsmaschine eingespannt. In dieser Maschine wechseln sich während des Beschichtungsvorganges Standphasen, von 15 bis 45 Minuten, mit Rotationsphasen, von 1 bis 10 Minuten ab. Die Rotationsgeschwindigkeiten liegen zwischen 1 und 10 Umdrehungen pro Minute. Die Dauer der Standphasen, Bewegungsphasen und die Rotationsgeschwindigkeiten können in Abhängigkeit von der verwendeten Zellart, der Zelldichte und der gewünschten Zellschichtdicke variiert werden. Die Standphasen ermöglichen ein Absetzen der Zellen auf dem Substrat und geschieht entsprechend der Schwerkraft. Während der Rotationsphasen werden noch nicht adhärente Zellen wieder in Suspension gebracht und im Inkubator verteilt. Die Rotationsgeschwindigkeit kann so eingestellt werden, dass nach jeder Umdrehung ein anderer Teil der Implantatoberfläche nach oben ausgerichtet ist und somit für die Zellbesiedelung exponiert wird. Dies wird dadurch ermöglicht, dass die jeweils letzte Umdrehung eines Rotationszyklus nicht vollständig durchgeführt wird, sondern nur 331 bis 359° beträgt.

### Beispiel 2

### Verwendung der Besiedelungsvorrichtung zur Zellbesiedelung von Herzklappensegeln

Bei einer Herzklappenprothese sind die Klappensegel deutlich dünner als die entsprechende Aortenwand, so dass auch eine aufgesiedelte Zellschicht auf den Klappensegeln dünner sein muss. Auch sollen die Klappensegel beidseitig zellbesiedelt sein. Der wie im vorigen Beispiel 1 ausgelegte Inkubator trägt die Vorform der zu besiedelnden Herzklappenprothese im Inlay fixiert. Das Füllvolumen beträgt 100 ml, einschließlich 10 ml Zellsuspension mit darin enthaltenen 10⁶ bis 10⁷ Zellen. Die Inkubationszeit beträgt 12 bis 24 Stunden, die Rotationsphasen 1 bis 10 Minuten, die Ruhephasen 15 bis 45 Minuten. Zusätzlich wird während der Inkubation die Vorrichtung im Uhrzeigersinn gedreht, wodurch sich das Inlay bei jeder Drehung um 180° bewegt und es bei jeder Drehung um 360° zu einem kleinen Strom durch die Klappensegel mit entsprechenden Bewegungen derselben kommt. Durch diese Bewegung wird erreicht, dass es zu einem Austausch der Zellsuspension oberhalb und unterhalb der Segel kommt, wodurch die Zellbesiedelung homogener wird. Wie im Beispiel 1 enden auch hier die Rotationen des Inkubators immer in einer anderen Position, so dass in jeder Ruhephase ein anderer Teil der Oberfläche exponiert ist.

### Beispiel 3

### Zellbesiedelung einer Gefäßprothese - Variante 1

Unter Verwendung der im obigen Beispiel 1 beschriebenen Besiedelungsvorrichtung wird ein als Gefäßprothese ausgebildetes Implantat zunächst mit Fibroblasten Anschließend folgt eine Ruheperiode von 2 bis 8 Tagen in Kulturmedium, um das Anwachsen der Zellen und ihre Haftung sicherzustellen. Dies ist abhängig von der initialen Beschichtungsdichte und der Anhaftung an der Prothese. Nach dieser Phase wird dann mit Endothelzellen besiedelt. Nach einer weiteren Ruhephase von 2 bis 7 Tagen ist die Prothese zur Implantation in den Patienten einsatzbereit.

### Beispiel 4

### Zellbesiedelung einer Gefäßprothese - Variante 2

Unter Verwendung der im obigen Beispiel 1 beschriebenen Besiedelungsvorrichtung wird ein als Gefäßprothese ausgebildetes Implantat zunächst mit einer Mischkultur aus glatten Muskelzellen und Fibroblasten besiedelt. Anschließend folgt eine Ruheperiode von 2 bis 8 Tagen in Kulturmedium, um das Anwachsen der Zellen und die Haftung sicherzustellen. Nach dieser Phase wird dann mit Endothelzellen besiedelt. Nach einer weiteren Ruhephase von 2 bis 7 Tagen ist die Prothese zur Implantation in den Patienten einsatzbereit.

### Beispiel 5

### Zellbesiedelung einer Aortenklappe - Variante 1

Analog dem Verfahren von Beispiel 3 wird bei einer Vorform eines prothetischen Aortenrohrs die Außen- und Innenwand mit Fibroblasten (Zellsuspension mit > 10⁷ Zellen) mittels des Inkubators von Beispiel 1 beschichtet. Nach einer Ruhephase von einem Tag wird mit der Vorrichtung von Beispiel 2 und einer geringeren Zellzahl von 10⁵ bis 10⁶ Zellen erneut mit Fibroblasten beschichtet, um auch auf den Klappensegeln ein entsprechendes Zell-Layer zu erzeugen. Hieran schließt sich eine Ruhephase von 2 bis 8 Tagen an. Danach wird im Inkubator von Beispiel 2 die Aortenprothese mit Endothelzellen besiedelt. Nach einer weiteren Ruhephase von 2 bis 7 Tagen ist die Prothese dann zur Implantation in den Patienten einsatzbereit.

### Beispiel 6

### Zellbesiedelung einer Aortenklappe - Variante 2

Analog dem Verfahren von Beispiel 3 wird bei einer Vorform eines prothetischen Aortenrohrs die Aussen- und Innenwand mit einer Mischkultur aus Fibroblasten und glatten Muskelzellen (> 10⁷ Zellen) im Inkubator von Beispiel 1 besiedelt. Nach einer Ruhephase von einem Tag wird mit dem Inkubator von Beispiel 2 und einer geringeren Zellzahl (10⁵ bis 10⁶ Zellen) erneut mit Fibroblasten beschichtet, um auch auf den Segeln ein entsprechendes Zell-Layer zu erzeugen. Hieran schließt sich eine Ruhephase von 2 bis 8 Tagen an. Danach wird im Inkubator von Beispiel 2 mit Endothelzellen besiedelt. nach einer weiteren Ruhephase von 2 bis 7 Tagen ist die Prothese dann zur Implantation in den Patienten einsatzbereit.

### Beispiel 7

### Ausbildung einer Herzklappenprothese

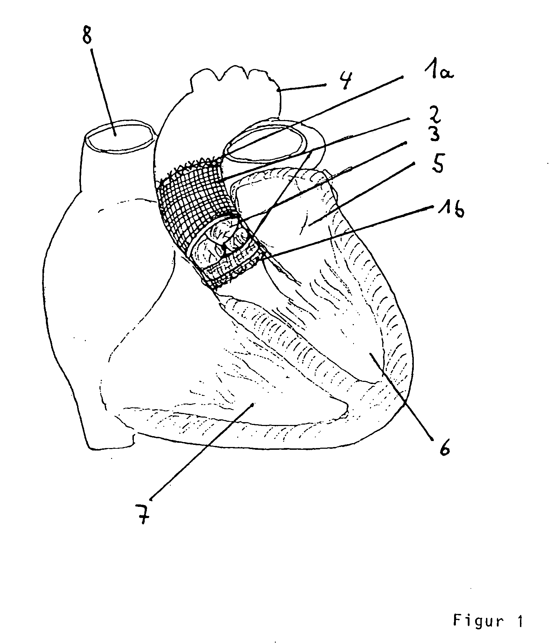

Zur Erläuterung des Ausführungsbeispiels wird auf die beigefügte Figur 1 Bezug genommen, die eine Herzklappenprothese zeigt, die zur besseren Übersicht teilweise geöffnet ist. In der Zeichnung bezeichnen die Bezugszeichen 1 a/b Nahtringe, 2 Aortenstumpfprothese, 3 Arterienklappenprothese, 4 Aorta, 5 linker Vorhof, 6 linke Herzkammer, 7 rechte Herzkammer und 8 obere Hohlvene.

Das Implantat wird gebildet mit Polyurethanvlies als Segel und gewebter/gewirkter Gefäßprothese aus Polyester. Eine gewebte Aortenstumpfprothese (2) wurde auf einen Formkern gespannt, der den Gewebeschlauch offen hält und ein Abbild der Innenkonturen einer Herzklappe mit Aortenstumpf und geschlossenen Segeln darstellt. Durch Versprühen einer Polyurethanlösung auf den Schlauch und den im Schlauch befindlichen Formkern, kann die Herzklappenprothese (3) direkt in die Aortenstumpfprothese integriert werden. Es kann so ein Durchdringen der feinen (delikaten) Vliesstruktur, die die Herzklappenprothese bildet und mit dem Aortenstumpf verbindet, in die gewebte Aortenstumpfprothese erzielt werden. Die Nahtringe 1 a und 1 b werden aus einem aus Polyurethanlösung gesprühten, dann aufgerollten Vliesschlauch gebildet. Nach dem Herausnehmen des Formkerns werden die Segel durch Einschnitte voneinander getrennt. Sie verbleiben in unbelastetem Zustand in der Schließstellung.

## Patentansprüche

1. Kardiovaskuläres Implantat zur Verwendung in der Chirurgie, insbesondere Herzklappe mit Segeln, gebildet im Wesentlichen aus bioverträglichem synthetischem Polymermaterial in Form einer dreidimensionalen Struktur, **dadurch gekennzeichnet, dass** das Polymermaterial unter physiologischen Bedingungen im Wesentlichen nicht resorbierbar ist, das Implantat mindestens an einem Teil seiner Oberfläche als mikroporöses Vlies aus Mikrofasern des Polymermaterials gebildet ist, die eine Besiedelung mit Zellen ermöglichen.

2. Implantat nach einem der vorhergehenden Ansprüche, **dadurch gekennzeichnet, dass** das Vlies eine Dicke von 10 bis 500 µm, insbesondere 30 bis 70 µm aufweist.

3. Implantat nach einem der vorhergehenden Ansprüche, **dadurch gekennzeichnet, dass** die Mikrofasern eine Dicke von 0,1 bis 10 µm aufweisen.

4. Implantat nach einem der vorhergehenden Ansprüche, **dadurch gekennzeichnet, dass** das Vlies eine Porengröße im Bereich von 0,1 bis 20 µm, insbesondere 1 bis 8 µm aufweist.

5. Implantat nach einem der vorhergehenden Ansprüche in Form einer Herzklappenprothese umfassend einen rohrförmigem Abschnitt als Stumpf eines Herzblutgefäßes, insbesondere Aortenstumpf, der zum Annähen an ein Endstück eines natürlichen Blutgefäßes ausgebildet ist, wobei an den Stumpf drei eine Herzklappe bildende Segel relativ zueinander beweglich angeordnet sind, und als Ventil miteinander zusammenwirken, um bei Blutdurchfluss zu öffnen und zu schließen.

6. Implantat nach einem der vorhergehenden Ansprüche, **dadurch gekennzeichnet, dass** die Herzklappensegel aus flächigem Vlies gebildet sind.

7. Implantat nach einem der vorhergehenden Ansprüche, **dadurch gekennzeichnet, dass** die Herzklappensegel gewölbt sind.

8. Implantat nach einem der vorhergehenden Ansprüche, **dadurch gekennzeichnet, dass** das Implantat im Bereich des Gefäßabschnitts eine Wanddicke von 1 bis 3 mm aufweist.

9. Implantat nach einem der Ansprüche 1 bis 8, **dadurch gekennzeichnet, dass** die Herzklappensegel als getrennte Teile hergestellt und am rohrförmigen Abschnitt der Prothese befestigt, insbesondere angeklebt oder angeschmolzen sind.

10. Implantat nach einem der Ansprüche 1 bis 8, **dadurch gekennzeichnet, dass** die Herzklappenprothese, insbesondere der rohrförmige Abschnitt mit den Segeln, einstückig ausgebildet ist.

11. Implantat nach einem der Ansprüche 5 bis 9, **dadurch gekennzeichnet, dass** die Herzklappensegel und der rohrförmige Abschnitt eine unterschiedliche Struktur aufweisen.

12. Implantat nach einem der Ansprüche 5 bis 11, **dadurch gekennzeichnet, dass** die Herzklappensegel und der rohrförmige Abschnitt aus unterschiedlichen Materialien hergestellt sind.

13. Implantat nach einem der vorhergehenden Ansprüche, **dadurch gekennzeichnet, dass** die Herzklappenprothese, insbesondere ihr rohrförmiger Abschnitt, mit einer Verstärkung, insbesondere in Form einer textilen Konstruktion versehen ist.

14. Implantat nach einem der vorhergehenden Ansprüche, **dadurch gekennzeichnet, dass** es mindestens teilweise nach einer Sprühvliestechnik hergestellt ist.

15. Implantat nach einem der vorhergehenden Ansprüche, dadurch insbesondere humanen Zellen ausgebildet ist.

16. Implantat nach einem der vorhergehenden Ansprüche, **dadurch gekennzeichnet, dass** es mindestens teilweise, insbesondere mehrschichtig, mit humanen Zellen besiedelt ist.

17. Implantat nach einem der vorhergehenden Ansprüche, **dadurch gekennzeichnet, dass** angesiedelte Zellen die dem Blut zugewandte Oberfläche als flächiger Zellrasen überziehen.

18. Implantat nach einem der vorhergehenden Ansprüche, **dadurch gekennzeichnet, dass** angesiedelte Zellen ausgewählt sind aus der Gruppe der Fibroblasten, glatten Muskelzellen und Endothelzellen.

19. Implantat nach einem der vorhergehenden Ansprüche, **dadurch gekennzeichnet, dass** die Endothelzellen auf einer Fibroblasten und/oder glatte Muskelzellen enthaltenden Zellschicht auf dem Implantat angesiedelt sind.

20. Implantat nach einem der Ansprüche 17 bis 19, **dadurch gekennzeichnet, dass** der angesiedelte Zellrasen eine hohe Scherfestigkeit aufweist.

21. Verfahren zur Herstellung eines biohybriden Implantats, insbesondere nach einem der Ansprüche 16 bis 20, gebildet im Wesentlichen aus bioverträglichem synthetischem Polymermaterial in Form einer dreidimensionalen Struktur mit vliesartiger Oberfläche, **dadurch gekennzeichnet, dass** eine Vorform des Implantats aus synthetischem Polymermaterial mit humanen Zellen besiedelt wird, wobei zunächst Fibroblasten und/oder glatte Muskelzellen, angesiedelt werden, dann vorzugsweise nach einer Ruhephase zur Verankerung der ersten Zellschicht, in einer Oberflächen-Zellschicht Endothelzellen angesiedelt werden.

22. Verfahren nach Anspruch 21, **dadurch gekennzeichnet, dass** Endothelzellen, Fibroblasten und glatte Muskelzellen aus explantierten Stücken der Vena saphena magna des zu behandelnden Patienten gewonnen und in spezifischen Zellmedien unter Zusatz von humanem Serum des Patienten kultiviert werden.

23. Verfahren nach Anspruch 22, **dadurch gekennzeichnet, dass** die Herzklappe während und/oder nach ihrer Herstellung mit rohr- bzw. schlauchartigen Verbindungsstücken verbunden wird, die zum Verbinden mit dem Herzen und dem Aortenbogen dienen.

24. Vorrichtung zur Herstellung eines biohybriden Implantats, insbesondere zur Durchführung des Verfahrens nach einem der Ansprüche 21 bis 23, **gekennzeichnet durch** einen zur Zellbesiedelung einer Vorform eines chirurgischen Implantats aus synthetischem Polymermaterial eingerichteten Inkubator zur Aufnahme des Zellmediums, insbesondere einer Zellsuspension, der um mindestens eine Achse drehbar ist, und ein darin aufnehmbares Gestell zur Aufnahme und Fixierung des mit Zellen zu beschichtenden Implantats aufweist.

25. Bereitstellung des Implantats nach einem der Ansprüche 1 bis 20 als Gefäßprothese in der kardiovaskulären Chirurgie.

26. Bereitstellung des Implantats nach einem der Ansprüche 1 bis 20 als Herzklappenprothese in der Herzchirurgie.
